# EUROPÄISCHE PATENTSCHRIFT

(11) **EP 4 154 864 B1**
(45) Veröffentlichungstag und Bekanntmachung des Hinweises auf die Patenterteilung: **17.12.2025**
(21) Anmeldenummer: 22197130.2
(22) Anmeldetag: 22.09.2022
(51) Int. Cl.: A61K 8/41, A61K 8/49, A61Q 5/10

(54) **MITTEL ZUR REPIGMENTIERUNG VON KERATINFASERN, INSBESONDERE MENSCHLICHEN HAAREN**
REPIGMENTING COMPOSITION FOR KERATIN FIBRES, ESPECIALLY HUMAN HAIR
COMPOSITION POUR LA REPIGMENTATION DES FIBRES KÉRATINIQUES, EN PARTICULIER DES CHEVEUX HUMAINS

(43) Veröffentlichungstag der Anmeldung: 29.03.2023
(60) Teilanmeldung: 24199211.4
(73) Patentinhaber: Dr. Kurt Wolff GmbH & Co. KG, 33611 Bielefeld (DE)
(72) Erfinder: Schulze zur Wiesche, Erik, 33611 Bielefeld (DE); Hagenstein, Miriam, 33611 Bielefeld (DE)
(74) Vertreter: Weickmann & Weickmann PartmbB

(56) Entgegenhaltungen:
- EP-B1- 0 971 682
- DE-A1- 102007 040 313
- DE-A1- 4 137 971
- US-B1- 6 423 100

## Beschreibung

Die vorliegende Erfindung betrifft eine kosmetische Formulierung enthaltend ein Farbstoffvorprodukt, ausgewählt aus der Gruppe bestehend aus Indol-Derivaten und Indolin-Derivaten, ein Xanthin-Derivat und ein direktziehender Farbstoff. Die Erfindung betrifft ferner die Verwendung der kosmetischen Formulierung zur Repigmentierung von Keratinfasern, insbesondere menschlichen Haaren.

Die Haarfarbe eines Menschen hängt von der Menge der Melanine Eumelanin und Phäomelanin ab, die in der Faserschicht der Haare enthalten sind und von Melanozyten in den Haarfollikeln produziert werden. Im Rahmen des normalen Alterungsprozesses, aber auch bedingt durch bestimmte Krankheitszustände, Stress, Vitamin- und Mineralstoffmangel oder übermäßigen Alkohol- oder Nikotinkonsum kann es zum Nachlassen der Melaninproduktion kommen, in deren Folge es zu einer Hypopigmentierung kommt. Solche Haare erscheinen dem Betrachter weiß oder farblos. Der optische Eindruck von grauem Haar resultiert aus der Mischung aus pigmentierten und pigmentlosen Haaren.

Der durchschnittliche Lebenszyklus eines Haars beträgt drei bis sieben Jahre, dann fallen die Haare aus und ein neues Haar wächst an dieser Stelle. Allmählich überwiegt der Anteil an weißen oder pigmentlosen Haaren, das Haar ergraut.

Die Veränderung der Haarfarbe und insbesondere die Pigmentierung oder Repigmentierung von grauen Haaren stellt einen wichtigen Bereich der modernen Kosmetik dar. Hierbei sind insbesondere gute Echtheitseigenschaften und gute Grauabdeckung sowie langanhaltende Dauer der Färbungen wünschenswert.

Für permanente, intensive Färbungen mit guten Echtheitseigenschaften und guter Grauabdeckung werden herkömmlicherweise Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln, wie z.B. Wasserstoffperoxid, oder von Luftsauerstoff untereinander oder unter Kupplung mit einer oder mehreren Kupplerkomponenten die eigentlichen Farbstoffe ausbilden.

Der Einsatz oxidativer Färbemittel ist jedoch nach wie vor mit Beeinträchtigungen oder Schädigungen der Haare und insbesondere deren Oberflächenstruktur verbunden.

Alternativ können Vorstufen des natürlichen Haarfarbstoffes Melanin auf das Haar aufgebracht werden, die im Rahmen oxidativer Prozesse im Haar praktisch naturidentische Farbstoffe ausbilden. Die Ausfärbung kann dabei mit Luftsauerstoff als einzigem Oxidationsmittel erfolgen, so dass keine weiteren Oxidationsmittel erforderlich sind. Ein solches Verfahren mit 5,6-Dihydroxyindolin als Farbstoffvorprodukt ist beispielsweise in EP 0 530 229 B1 beschrieben.

Im Gegensatz dazu kommen für temporäre Färbungen direktziehende Farbstoffe, auch Direktzieher genannt, zum Einsatz. Hierbei handelt es sich um Farbstoffmoleküle, die direkt auf das Haar aufziehen und keinen oxidativen Prozess zur Ausbildung der Farbe mehr benötigen. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

WO 99/66890 beschreibt ein Färbemittel zum Färben von Keratinfasern enthaltend ein Farbstoffvorprodukt, bei welchem es sich um ein Indolin- oder Indol-Derivat, z.B. ein Derivat des 5,6-Dihydroxyindolins oder des 5,6-Dihydroxyindols, handeln kann, sowie eine Aminosäure oder ein Oligopeptid. Die Ausbildung der Farbe kann mittels Luftoxidation erfolgen.

DE 10 2018 127 182 A1 betrifft ein Zweikomponentensystem zur künstlichen Haarfärbung enthaltend getrennt voneinander ein wasserfreies Trägermedium, welches ein Alkan, einen Fettalkohol und einen Alkohol umfasst, sowie eine wässrige Phase, welche Wasserstoffperoxid enthält. Eine siliciumorganische Verbindung aus der Gruppe der Silane kann zum Schutz und zur Pflege der Haare enthalten sein.

DE 10 2007 038 484 A1 beschreibt ein Haarbehandlungsmittel zum Schutz vor äußeren Einflüssen, wobei das Haarbehandlungsmittel Bakterienferment(e) aus *Thermus thermophilus* enthält.

WO 2005/007615 A1 betrifft 2-(Amino- oder substituierte Amino)-5-(substituierte oxymethyl)-phenolverbindungen sowie Zusammensetzungen zum oxidativen Färben von Keratinfasern.

DE102007040313 handelt von Haarbehandlungsmitteln zur Haarkräftigung, die eine Kombination aus einer Biotinverbindung, einer Taurinverbindung, einer Purinverbindung wie Koffein, und einem Glykoprotein, enthalten. Das Präparat kann zusätzlich eine farbverändernde Komponente enthalten.

Mit den im Stand der Technik bekannten Formulierungen lassen sich jedoch keine ausreichend zufriedenstellenden Ergebnisse hinsichtlich Qualität, Intensität und Dauer der Färbeleistung bei gleichzeitig möglichst geringer Beeinträchtigung oder Schädigung der Haarstruktur erzielen.

So bilden sich mit den Vorstufen des natürlichen Haarfarbstoffes Melanin die entstehenden Farbkörper stark zeitverzögert und die Farbtöne lassen sich unzureichend kontrollieren. Zudem fallen die Zwischenfarbtöne oft unnatürlich violett aus. Ein weiterer Nachteil ist der mit dem Nachwachsen der Haare in Erscheinung tretende unpigmentierte, Haaransatz.

Zusammenfassend besteht insbesondere bei den Echtheitseigenschaften, der Grauabdeckung und der Dauer der Färbeleistung weiterhin Verbesserungsbedarf.

Ausgehend hiervon war es die Aufgabe der vorliegenden Erfindung, eine kosmetische Formulierung bereitzustellen, die zur Pigmentierung oder Repigmentierung von Keratinfasern, insbesondere menschlichen Haaren geeignet ist und insbesondere verbesserte Echtheitseigenschaften der pigmentierten oder repigmentierten Keratinfasern sowie eine verbesserte Grauabdeckung bietet. Weiterhin sollte diese kosmetische Formulierung hinsichtlich Qualität, Intensität und Dauer der Färbeleistung verbesserte Eigenschaften gegenüber aus dem Stand der Technik bekannten Formulierungen aufweisen.

Diese Aufgabe wurde überraschend durch die Formulierung gemäß Anspruch 1 und deren Verwendung gemäß Anspruch 5 ++ gelöst. Bevorzugte Ausführungsformen gehen aus den abhängigen Ansprüchen hervor.

Erfindungsgemäß wird die Aufgabe durch die Bereitstellung einer kosmetischen Formulierung gelöst, welche ein Farbstoffvorprodukt, ausgewählt aus der Gruppe bestehend aus den in Anspruch 1 definierten Indol-Derivaten und Indolin-Derivaten, das Xanthin-Derivat Koffein und mindestens einen direktziehenden Farbstoff enthält.

Es wurde überraschenderweise festgestellt, dass der Kontrast zum nachwachsenden Haar (Haaransatz) signifikant geringer ausfällt, da die natürliche Pigmentbildung in der Haarwurzel angeregt wird, bei gleichzeitiger Verdunkelung des bereits herausgewachsenen Haares.

Insgesamt werden die Haare schrittweise dunkler, wobei sich der Farbeffekt dezent aufbaut, graue Haare deutlich abgedeckt werden, der Gesamteindruck der Haarfarbe deutlich dunkler wirkt.

Somit werden mit der erfindungsgemäßen kosmetischen Formulierung eine gute Grauabdeckung sowie ausgezeichnete Echtheitseigenschaften bei langanhaltender Dauer der Färbeleistung und einem reduzierten Nachgrauen des Haaransatzes erreicht.

Durch das in der erfindungsgemäßen Formulierung enthaltene Xanthin-Derivat kommt es zusammen mit dem Farbstoffvorprodukt zu einer Anregung der Keratinozyten in den Haarwurzeln, damit diese wieder verstärkt natürliches Pigment produzieren. Ferner bewirkt das Xanthin-Derivat einen längeren Verbleib des Haares auf dem Kopf. Somit wird zum einen der Verlust noch pigmentierter Haare verzögert und zum anderen ein Beitrag zur natürlichen Repigmentierung bereits pigmentarmer oder pigmentloser Haare geleistet. Im Rahmen der vorliegenden Erfindung werden die Begriffe Pigmentierung und Repigmentierung austauschbar verwendet.

Das in der erfindungsgemäßen Formulierung enthaltene Farbstoffvorprodukt, bei dem es sich um ein Indol-Derivat oder Indolin-Derivat handelt, ist in Anspruch 1 definiert.

In einer bevorzugten Ausführungsform der Erfindung handelt es sich bei dem Farbstoffvorprodukt um ein Indol-Derivat.

Erfindungsgemäß bevorzugt ist das Farbstoffvorprodukt ein Derivat des 5,6-Dihydroxyindols der Formel (I), 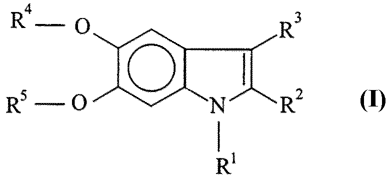 wobei, jeweils unabhängig voneinander,
R¹ Wasserstoff, eine C₁-C₄-Alkylgruppe oder eine C₁-C₄-Hydroxyalkylgruppe bedeutet,
R² Wasserstoff oder eine-COOH-Gruppe bedeutet, wobei die-COOH-Gruppe auch als Salz mit einem physiologisch verträglichen Kation vorliegen kann,
R³ Wasserstoff oder eine C₁-C₄-Alkylgruppe bedeutet,
R⁴ Wasserstoff, eine C₁-C₄-Alkylgruppe, eine Aminogruppe oder eine Gruppe -CO-R⁶ bedeutet, in der R⁶ eine C₁-C₄-Alkylgruppe bedeutet, und
R⁵ eine der unter R⁴ genannten Gruppen bedeutet,
oder ein physiologisch verträgliches Salz dieser Verbindungen mit einer organischen oder anorganischen Säure.

Erfindungsgemäß bevorzugte Vertreter sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol.

In einer besonders bevorzugten Ausführungsform ist die Verbindung der Formel (I) ausgewählt aus 5,6-Dihydroxyindol, N-Methyl-5,6-dihydroxyindol sowie deren physiologisch verträglichen Salzen.

Ganz besonders bevorzugt handelt es sich bei dem in der erfindungsgemäßen Formulierung eingesetzten Farbstoffvorprodukt um 5,6-Dihydroxyindol.

In einer weiteren bevorzugten Ausführungsform der Erfindung handelt es sich bei dem Farbstoffvorprodukt um ein Indolin-Derivat.

Erfindungsgemäß bevorzugt ist das Farbstoffvorprodukt ein Derivat des 5,6-Dihydroxyindolins der Formel (II), 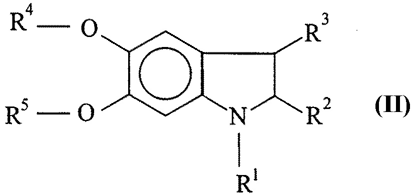 wobei, jeweils unabhängig voneinander,
R¹ Wasserstoff, eine C₁-C₄-Alkylgruppe oder eine C₁-C₄-Hydroxyalkylgruppe bedeutet,
R² Wasserstoff oder eine-COOH-Gruppe bedeutet, wobei die-COOH-Gruppe auch als Salz mit einem physiologisch verträglichen Kation vorliegen kann,
R³ Wasserstoff oder eine C₁-C₄-Alkylgruppe bedeutet,
R⁴ Wasserstoff, eine C₁-C₄-Alkylgruppe, eine Aminogruppe oder eine Gruppe -CO-R⁶ bedeutet, in der R⁶ eine C₁-C₄-Alkylgruppe bedeutet, und
R⁵ eine der unter R⁴ genannten Gruppen bedeutet,
oder ein physiologisch verträgliches Salz dieser Verbindungen mit einer organischen oder anorganischen Säure.

Erfindungsgemäß bevorzugte Vertreter sind 5,6-Dihydroxyindolin, N-Methyl-5,6-dihydroxyindolin, N-Ethyl-5,6-dihydroxyindol.

In einer besonders bevorzugten Ausführungsform ist die Verbindung der Formel (II) ausgewählt aus 5,6-Dihydroxyindolin, N-Methyl-5,6-dihydroxyindolin sowie deren physiologisch verträglichen Salzen.

Ganz besonders bevorzugt handelt es sich bei dem in der erfindungsgemäßen Formulierung eingesetzten Farbstoffvorprodukt um 5,6-Dihydroxyindolin.

In einer weiteren besonders bevorzugten Ausführungsform handelt es sich bei dem in der erfindungsgemäßen Formulierung eingesetzten Farbstoffvorprodukt um Dihydroxyindolin-hydrobromid (2,3-Dihydro-1H-indol-5,6-diol-hydrobromid).

Die in den erfindungsgemäßen Mitteln enthaltenen Indol- und Indolin-Derivate können sowohl als freie Basen als auch in Form ihrer physiologisch verträglichen Salze mit anorganischen oder organischen Säuren, z. B. der Hydrochloride, der Sulfate und Hydrobromide, eingesetzt werden.

Das Indol- oder Indolin-Derivat ist in der erfindungsgemäßen Formulierung üblicherweise in einer Menge von 0,001-5 Gew.-%, vorzugsweise 0,005-2 Gew.-%, bevorzugt 0,01-2 Gew.-%, bevorzugt 0,01-1,5 Gew.-%, bevorzugt in einer Menge von etwa 0,05 Gew.-%, 0,1 Gew.-%, 0,2 Gew.-%, 0,3 Gew.-%, 0,4 Gew.-%, 0,5 Gew.-%, 0,6 Gew.-%, 0,7 Gew.-%, 0,8 Gew.-%, 0,9 Gew.-%, 1,0 Gew.-%, 1,5 Gew.-% jeweils bezogen auf das Gesamtgewicht der Formulierung, enthalten.

In einer besonders bevorzugten Ausführungsform handelt es sich bei dem Farbstoffvorprodukt um ein Indol-Derivat, vorzugsweise 5,6-Dihydroxyindol, wobei dieses in der erfindungsgemäßen Formulierung in einer Menge von 0,01-2 Gew.-%, bevorzugt 0,05-0,6 Gew.-%, bevorzugt in einer Menge von 0,08-0,5 Gew.-%, bevorzugt in einer Menge von etwa 0,1-0,5 Gew.-%, jeweils bezogen auf das Gesamtgewicht der Formulierung, enthalten ist.

In einer weiteren besonders bevorzugten Ausführungsform handelt es sich bei dem Farbstoffvorprodukt um ein Indolin-Derivat, vorzugsweise Dihydroxyindolin-hydrobromid (2,3-Dihydro-1H-indol-5,6-diol-hydrobromid), wobei dieses in der erfindungsgemäßen Formulierung in einer Menge von 0,01-4,0 Gew.-%, bevorzugt 0,05-2,0 Gew.-%, bevorzugt in einer Menge von 0,08-1,0 Gew.-%, bevorzugt in einer Menge von etwa 0,1-0,5 Gew.-%, jeweils bezogen auf das Gesamtgewicht der Formulierung, enthalten ist.

Die erfindungsgemäße Formulierung kann auch mehr als ein Indol- oder Indolin-Derivat oder Mischungen von Indol- und Indolin-Derivaten enthalten.

Die Ausbildung des Pigments aus dem Farbstoffvorprodukt, d. h. dem Indol- oder Indolin-Derivat, erfolgt durch Reaktion mit Luftsauerstoff, es ist kein zusätzliches Oxidationsmittel erforderlich.

Das in der kosmetischen Formulierung enthaltene Xanthin-Derivat führt zusammen mit dem Farbstoffvorprodukt insbesondere dazu, dass die Keratinozyten in den Haarwurzeln angeregt werden, wieder verstärkt natürliches Pigment zu produzieren. Darüber hinaus bewirkt das Xanthin-Derivat einen längeren Verbleib des Haares auf dem Kopf, was zum einen den Verlust noch pigmentierter Haare verzögert und zum anderen zur natürlichen Repigmentierung bereits pigmentarmer oder pigmentloser Haare beiträgt.

Das erfindungsgemäß Xanthin-Derivat ist Koffein.

Der IUPAC-Name von Koffein ist 1,3,7-Trimethyl-3,7-dihydro-1H-purin-2,6-dion. Alternativ wird Koffein auch als 1,3,7-Trimethylxanthin bezeichnet. Koffein wird durch die folgende chemische Formel (III) dargestellt.

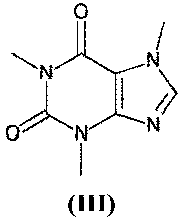

Koffein ist ein zur Klasse der Methylxanthine gehörendes Methylxanthinalkaloid. Es ist eine bittere kristalline Substanz, kann als Purinderivat betrachtet werden und ist chemisch mit den Adenin- und Guaninbasen der Desoxyribonukleinsäuren und der Ribonukleinsäure verwandt.

Das Xanthin-Derivat ist in der erfindungsgemäßen Formulierung üblicherweise in einer Menge von 0,0001-10 Gew.-%, bevorzugt 0,01-5 Gew.-%, bevorzugt 0,05-2 Gew.-%, bevorzugt 0,1-1,5 Gew.-%, bevorzugt in einer Menge von etwa 0,0001 Gew.-%, 0,05 Gew.-%, 0,1 Gew.-%, 0,2 Gew.-%, 0,3 Gew.-%, 0,5 Gew.-%, 0,7 Gew.-%, 1,0 Gew.-%, 1,3 Gew.-%, 1,4 Gew.-%, 1,5 Gew.-%, jeweils bezogen auf das Gesamtgewicht der Formulierung, enthalten.

Die erfindungsgemäßen Formulierungen enthalten mindestens einen direktziehenden Farbstoff.

Hierbei wurde überraschenderweise festgestellt, dass der Farbeffekt einer Formulierung, die neben dem Farbstoffvorprodukt vom Indol- oder Indolin-Typ und dem Xanthin-Derivat auch noch einen direktziehenden Farbstoff enthält, intensiver ist, als anhand der Farbeffekte der Einzelkomponenten, Farbstoffvorprodukt vom Indol- oder Indolin-Typ und direktziehender Farbstoff, zu erwarten gewesen wäre. Die Verdunklungseffekte (Repigmentierung) werden durch die Kombination mit direktziehenden Farbstoffen synergistisch bezüglich der Schnelligkeit des Farbeintritts und der Farbtiefe gesteigert.

Bei Anwesenheit einer Kombination aus Farbstoffvorprodukt vom Indol- oder Indolin-Typ und direktziehendem Farbstoff tritt somit ein synergistischer Effekt auf. Somit wird mit der erfindungsgemäßen kosmetischen Formulierung enthaltend ein Farbstoffvorprodukt vom Indol- oder Indolin-Typ, ein Xanthin-Derivat und einen direktziehenden Farbstoff vorteilhafterweise ein besonders intensiver Farbeffekt erreicht, wobei eine hervorragende Grauabdeckung sowie ausgezeichnete Echtheitseigenschaften, d. h. ein in hohem Maße natürlicher Farbton bei langanhaltender Dauer des Färbeergebnisses erreicht wird.

Wie eingangs erwähnt, handelt es sich bei direktziehenden Farbstoffen um Farbstoffmoleküle, die direkt auf das Haar aufziehen und keinen oxidativen Prozess zur Ausbildung der Farbe mehr benötigen.

Bevorzugte direktziehende Farbstoffe umfassen Nitrophenylendiamine, Nitroaminophenole, Azofarbstoffe, Anthrachinone und Indophenole und sind vorzugsweise ausgewählt aus der Gruppe der unter den internationalen Bezeichnungen bzw. Handelsnamen bekannten Farbstoffe HC Yellow 2, HC Yellow 4, HC Yellow 5, HC Yellow 6, HC Yellow 12, Acid Yellow 1, Acid Yellow 10, Acid Yellow 23, Acid Yellow 36, Basic Yellow 57, Basic Yellow 87, HC Orange 1, Disperse Orange 3, Acid Orange 7, Basic Orange 31, HC Red 1, HC Red 3, HC Red 10, HC Red 11, HC Red 13, HC Red BN, Acid Red 33, Acid Red 52, Pigment Red 57:1, Basic Red 51, Basic Red 76, HC Blue 2, HC Blue 12, HC Blue 16, Disperse Blue 3, Acid Blue 7, Basic Blue 99, Acid Green 50, HC Violet 1, Disperse Violet 1, Disperse Violet 4, Acid Violet 43, Disperse Black 9, Acid Black 1, Acid Black 52, Basic Brown 16 und Basic Brown 17 sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxyethyl)amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Hydroxyethyl-2-nitro-toluidin, Pikraminsäure und deren Salze, 2-Amino-6-chlor-4-nitrophenol, 4-Ethylamino-3-nitrobenzoesäure und 2-Chlor-6-ethylamino-1-hydroxy-4-nitrobenzol.

Der direktziehende Farbstoff ist in der erfindungsgemäßen Formulierung üblicherweise in einer Menge von 0,001-5 Gew.-%, vorzugsweise 0,05-3 Gew.-%, bevorzugt 0,1-2 Gew.-%, bevorzugt 0,1-1,2 Gew.-%, bevorzugt in einer Menge von etwa 0,1 Gew.-%, 0,2 Gew.-%, 0,3 Gew.-%, 0,4 Gew.-%, 0,5 Gew.-%, 0,6 Gew.-%, 0,7 Gew.-%, 0,8 Gew.-%, 0,9 Gew.-%, 1,0 Gew.-%, 1,1 Gew.-%, 1,2 Gew.-%, 1,3 Gew.-%, 1,4 Gew.-%, 1,5 Gew.-%, 1,6 Gew.-%, 1,7 Gew.-%, 1,8 Gew.-%, 1,9 Gew.-%, 2,0 Gew.-%, jeweils bezogen auf das Gesamtgewicht der Formulierung, enthalten.

In einer bevorzugten Ausführungsform ist der direktziehende Farbstoff ausgewählt aus der Gruppe bestehend aus 2-Amino-6-chlor-4-nitrophenol, 1,4-Bis-(β-hydroxyethyl)-amino-2-nitrobenzol, Basic Yellow 57, Basic Red 76, Basic Brown 16, Basic Brown 17, Basic Blue 99, HC Blue 2, HC Blue 12, HC Blue 16 und Acid Violet 43.

In einer besonders bevorzugten Ausführungsform der Erfindung handelt es sich bei dem direktziehenden Farbstoff um HC Blue 2.

In einer weiteren besonders bevorzugten Ausführungsform enthält die erfindungsgemäße Formulierung 5,6-Dihydroxyindol als Farbstoffvorprodukt, Koffein als Xanthin-Derivat und ferner HC Blue 2 als direktziehenden Farbstoff. Hierbei ist 5,6-Dihydroxyindol in einer Menge von 0,05-0,6 Gew.-%, Koffein in einer Menge von 0,8-1,2 Gew.-% und der direktziehende Farbstoff in einer Menge von 0,1-1,2 Gew.-%, jeweils bezogen auf das Gesamtgewicht der Formulierung, enthalten.

Weiterhin kann die erfindungsgemäße kosmetische Formulierung alle in solchen Zubereitungen bekannten Additive (d. h. Wirk-, Zusatz- und Hilfsstoffe) enthalten.

In einer Ausführungsform umfasst die kosmetische Formulierung ein Tensid. Das Tensid kann ein anionisches, nichtionisches, kationisches oder zwitterionisches Tensid sein. Bevorzugt ist das Tensid ein anionisches oder nichtionisches Tensid, insbesondere ein möglichst mildes (d.h. besonders hautverträgliches) anionisches oder nichtionisches Tensid. Nichtionische Tenside werden dabei erfindungsgemäß insbesondere wegen ihrer sehr guten Emulgationseigenschaften sowie ihrer ausgezeichneten Hautpflegeeigenschaften eingesetzt. Anionische Tenside sind bevorzugt, weil sie eine besonders hohe Reinigungsleistung aufweisen. Daher eignen sie sich besonders gut in Reinigungsformulierungen wie Shampoos. Kationische Tenside besitzen hervorragende Haarpflegeeigenschaften und werden erfindungsgemäß insbesondere in Haarpflegeformulierungen eingesetzt wie in Conditionern, Shampoos und Kuren.

Die erfindungsgemäße Formulierung enthält Tenside vorzugsweise in einer Menge von 2 bis 40 Gew.-%, insbesondere von 5 bis 30 Gew.-%, bevorzugt von 7 bis 20 Gew.-%, besonders bevorzugt von 10 bis 17 Gew.-%, jeweils bezogen auf das Gesamtgewicht der Formulierung. Geeignete Mengen an Tensid lauten: 8 Gew.-%; 9 Gew.-%; 10 Gew.-%; 11 Gew.-%; 12 Gew.-%; 13 Gew.-%, 14 Gew.-%, 15 Gew.-%, 16 Gew.-%, 17 Gew.-%, 18 Gew.-%, 19 Gew.-%, 20 Gew.-%, 21 Gew.-%, 22 Gew.-%, 23 Gew.-%, 24 Gew.-%, 25 Gew.-%, jeweils bezogen auf das Gesamtgewicht der Formulierung. Besonders bevorzugt enthält die kosmetische Formulierung der Erfindung ein oder mehrere anionische Tenside in einer Menge von 0,1 bis 20 Gew.-%, bevorzugt von 1 bis 17 Gew.-% und besonders bevorzugt von 5 bis 15 Gew.-%, jeweils bezogen auf das Gesamtgewicht der Formulierung. Geeignete Mengen an anionischem Tensid lauten: 1 Gew.-%, 2 Gew.-%, 3 Gew.-%; 4 Gew.-%, 5 Gew.-%, 6 Gew.-%, 7 Gew.-%, 8 Gew.-%, 9 Gew.-%, 10 Gew.-%, 11 Gew.-%, 12 Gew.-%, 13 Gew.-%, 14 Gew.-%, 15 Gew.-%, 16 Gew.-%, 17 Gew.-%, 18 Gew.-%, 19 Gew.-%, 20 Gew.-%, jeweils bezogen auf das Gesamtgewicht der Formulierung. In diesen Mengen weisen die Tenside eine besonders hohe Reinigungsleistung auf und sind äußerst verträglich für Haut, Kopfhaut und Haare.

Die Tenside der vorliegenden Erfindung sind u.a. in dem Buch "Surfactants and interfacial phenomena", von Milton Rosen und Joy Kunjappu, John Wiley & Sons, Inc.-Verlag, 2012, 4. Auflage beschrieben.

In einer bevorzugten Ausführungsform ist das Tensid ein anionisches Tensid ausgewählt aus Alkylcarboxylaten, Alkylsulfonaten, Alkylsulfaten, Alkylethersulfaten, Alkylphosphaten, Alkylsarkosinaten, Alkyltauraten, Aminosäure-Tensiden und Mischungen davon. Besonders bevorzugt ist das Tensid ausgewählt aus Alkylcarboxylaten, Alkylsulfaten, Alkylsarkosinaten, Alkyltauraten, Alkylglutamat, wie Natriumcocoylglutamat/Dinatriumcocoylglutamat, Alkylgycinat, Alkylalaninat, wie Natriumcocoylalaninat und Mischungen davon. Ebenso bevorzugt wegen ihrer Reinigungsleistung sind Fettalkoholpolyglycerolethersulfate, Monoglyceridsulfate, Mono- und/oder Dialkylsulfosuccinate, Fettsäureisethionate, und α-Olefinsulfonate.

Alkylcarboxylate haben die generische Formel RCO₂M, Alkylsulfate haben die generische Formel ROSO₃M, Alkylsarkosinate haben die generische Formel RC(O)N(CH₃)CH₂CO₂M, und Alkyltaurate haben die generische Formel RC(O)N(CH₃)CH₂CH₂SO₃M, wobei R jeweils ein C₄-C₂₆-Alkyl oder C₄-C₂₆-Alkenyl ist und M ein wasserlösliches Kation wie z.B. Ammonium, Natrium oder Kalium darstellt. Vorzugsweise ist M ein Natriumkation. Bevorzugt ist Rein C₁₂-C₁₆-Alkyl oder ein C₁₂-C₁₈-Alkyl.

In einer Ausführungsform ist das Tensid ein nichtionisches Tensid (auch als nichtionischer Emulgator bezeichnet). Nicht-einschränkende Beispiele umfassen Glycerinfettsäureester, Fettalkohole, Polyoxyethylenether eines oder mehrerer Fettalkohole, alkoxylierte Fettsäurealkylester, Polyglycerinether von Fettalkoholen, Polyglycerinester von Fettsäuren, Polyethylenglykol- und/oder Polypropylenglykolether, Fettsäureamide, Alkylphenolpolyglycolether, Aminoxide und Alkylpolyglucoside.

In einer Ausführungsform ist das Tensid ausgewählt aus der Gruppe der Glycerinfettsäureester, Fettalkohole, Polyoxyethylenether eines oder mehrerer Fettalkohole, Polyglycerinether von Fettalkoholen, Polyglycerinester von Fettsäuren und deren Mischungen.

In der vorliegenden Erfindung bezieht sich der Begriff "Glycerinfettsäureester" auf einen Glycerinmono- oder Glycerindifettsäureester. Glycerindifettsäureester weisen die Formel R³-COO-(CH₂CH(OH)CH₂)-OOR⁴ oder R³-COO-(CH₂CH(OOR⁴)CH₂)-OH auf. Glycerinmonofettsäureester weisen die Formel R³-COO-(CH₂CH(OH)CH₂)-OH oder HO-(CH₂CH(OOR³)CH₂)-OH auf. Dabei sind R³ und R⁴ unabhängig voneinander aus C₆-C₂₈-Alkyl und C₆-C₂₈-Alkenyl ausgewählt. Glycerinmonofettsäureester enthalten eine Glycerin-Gruppe, welche über eine Esterbindung an eine einzige Fettsäure gebunden. Beispiele sind Glycerinmonostearat, Glycerinmonobehenat, Glycerinmonocaprylat, Glycerinmonocaprat und Glycerinmonolaurat.

Fettalkohole (gesättigt) sind Verbindungen der Formel H₃C-(CH₂)ₙ-CH₂-OH, wobei n eine ganze Zahl zwischen 4 und 20 ist, vorzugsweise eine ganze Zahl zwischen 6 und 16.

Polyoxyethylenether sind Verbindungen der Formel R⁵(OC₂H₃)ₙOH, wobei R⁵ ausgewählt ist aus C₆-C₂₈-Alkyl, C₆-C₂₈-Alkenyl, substituierte und unsubstituierte Phenoxy-Gruppen; und n eine ganze Zahl größer 1 ist. Vorzugsweise wird der Polyoxyethylenether eines oder mehrerer Fettalkohole aus der Gruppe Steareth-2, Steareth-21, Makrogol-Cetostearylether 12, Ceteareth-25, Makrogol-Cetostearylether 20 und Mischungen der vorgenannten Verbindungen ausgewählt. Noch bevorzugter ist der Polyoxyethylenether eine Verbindung ausgewählt aus der Gruppe von Ceteareth-25, Makrogol-Cetostearylether 20 und Mischungen der vorgenannten Verbindungen.

Der Begriff "Polyglycerinether von Fettalkoholen" bezieht sich auf eine Verbindung der Formel R⁶O-(C₃H₆O₂)ₙ-H, wobei R⁶ ein verzweigtes oder lineares C₆-C₂₈-Alkyl oder C₆-C₂₈-Alkenyl ist und n eine ganze Zahl größer als 1, vorzugsweise eine ganze Zahl von 2 bis 10, ist. Es wird bevorzugt, dass die Formulierung 0,01 bis 15,0 Gew.-%, 0,1 bis 10,0 Gew.-% oder 1 bis 5,0 Gew.-% Polyglycerinether enthält.

Der Begriff "Polyglycerinester von Fettsäuren" bezieht sich auf Verbindungen, die sowohl eine Polyglycerineinheit als auch mindestens eine C₆-C₂₆-Alkyl- oder C₆-C₂₆-Alkenylcarbonsäureeinheit enthalten. Diese Verbindungen können die Formel R⁷-R⁸-(C₃H₆O₂)ₙ-H aufweisen, wobei R⁷ ein C₆-C₂₆-Alkanoat- oder C₆-C₂₆-Alkenoat-Rest ist und R⁸ ein geeignetes Verbindungsmolekül oder eine direkte Bindung ist. Somit können die Polyglycerineinheit und die C₆-C₂₆-Alkyl- oder C₆-C₂₆-Alkenylcarbonsäureeinheit direkt durch eine Esterbindung verbunden sein oder eine Verbindungseinheit enthalten, die diese beiden Einheiten miteinander verbindet. Nicht einschränkende Beispiele für diese Gruppe sind Polyglyceryl-3-methylglucosedistearat, Polyglycerinpolycrinoleat, Polyglyceryl-Dimerat-Isostearat, Polyglyceryl-2-Laurat, Polyglyceryl-2-Sesquiisostearat, Polyglyceryl-3-Distearat (Cremophor GS 32), Polyglyceryl-3-Oleat, Polyglyceryl-3-Methylglykose-Distearat, Polyglyceryl-4-Caprat (Polyglycerolcaprat T2010190), Polyglyceryl-4-Diisostearat / Polyhydroxystearat / Sebacat (Isolan GPS) und Polyglyceryl-4-Isostearat.

In einer Ausführungsform umfasst das Tensid ein kationisches Tensid, wie beispielsweise ein quaternäres Tensid. Quaternäre Tenside enthalten mindestens ein N-Atom, das mit 4 Alkyl- oder Arylgruppen kovalent verbunden ist. Dies führt, unabhängig vom pH-Wert, zu einer positiven Ladung. Vorteilhaft sind Alkylbetain, Alkylamidopropylbetain und Alkylamidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

In einer weiteren Ausführungsform enthält die erfindungsgemäße Formulierung mindestens ein Additiv. Bevorzugt sind Additive, die üblicherweise in Shampoos, Conditionern und Emulsionen zur Behandlung der Haut, der Kopfhaut und der Haare eingesetzt werden. Das mindestens eine Additiv kann in einem Anteil von 0,01 Gew.-% bis 12,0 Gew.-%, mehr bevorzugt von 0,25 Gew.-% bis 10,0 Gew.-%, insbesondere von 1,0 bis 7,0 Gew.-% vorliegen.

Das mindestens eine Additiv kann ferner ausgewählt sein aus der Gruppe bestehend aus Haarkonditionierungsmitteln, Rückfettern, Konservierungsmitteln, Stabilisatoren, Duftstoffen, Antioxidantien, Rheologiemodifikatoren, Verdickungsmitteln, Pflegemitteln, Farbstoffen, Perlganzmitteln, Aufhellungsmitteln, Lösungsmitteln, festigenden Verbindungen, Antischuppenwirkstoffen, Vitaminen, Alkalisierungsmitteln bzw. pH-Stellmitteln und Kombinationen hiervon.

Haarkonditionierungsmittel können statische Aufladungen der Haare durch das Neutralisieren von elektrischer Ladung an ihrer Oberfläche verringern. Beispiele für Haarkonditionierungsmittel sind quartäre Ammoniumverbindungen.

Rückfetter, auch Rückfettungsmittel oder Überfettungsmittel genannt, sind lipophile Substanzen, die eine störende Auswirkung auf die epidermale Barrierefunktion verhindern können. Beispiele für Rückfetter sind Wollwachs, Squalen, flüssiges Paraffin, pflanzliche Öle, Silikone und Cetylpalmitat.

Konservierungsmittel sind Substanzen, die zur Konservierung verwendet werden, indem sie die Formulierung zersetzende Mikroorganismen abtöten und/oder deren Wachstum hemmen. Vorzugsweise können die Konservierungsmittel ausgewählt sein aus der Gruppe bestehend aus Benzoesäure, Benzoesäurederivaten, Sorbinsäure, Sorbinsäurederivaten, Salicylsäure, Salicylsäurederivaten, Phenoxyethanol, Parabenen und Kombinationen hiervon. In einer bevorzugten Ausführungsform werden Natriumbenzoat und/oder Kaliumsorbat als Konservierungsmittel in der erfindungsgemäßen Formulierung eingesetzt. Natriumbenzoat setzt in leicht saurem Milieu Benzoesäure und Kaliumsorbat Sorbinsäure frei. Beiden Säuren wird eine antimikrobielle Wirkung zugeschrieben.

Stabilisatoren können lichtempfindliche Komponenten gegenüber Strahlung schützen und sind bevorzugt UV-Absorber wie beispielsweise Benzophenonderivate.

Die Zugabe von Duftstoffen kann für einen angenehmen Geruch der Formulierung sorgen. Beispiele sind die dem Fachmann bekannten Parfums.

Ein Antioxidans oder Antioxidationsmittel ist eine chemische Verbindung, die eine Oxidation anderer Komponenten in der erfindungsgemäßen Formulierung verlangsamt oder gänzlich verhindert. Als Antioxidantien kommen beispielsweise Zitronensäure, Ascorbinsäure, Natriumsulfit und Butylhydroxyanisol in Frage.

Rheologiemodifikatoren und Verdickungsmittel können dabei helfen, die Applikationseigenschaften der erfindungsgemäßen Formulierung zu verbessern. Als Rheologiemodifikator und Verdickungsmittel kann die Zugabe von Kochsalz (Natriumchlorid) in Betracht gezogen werden. Durch die Zugabe von Kochsalz kann die Fließfähigkeit der erfindungsgemäßen Formulierung in gewissen Grenzen beeinflusst und auf das nötige Maß eingestellt werden. Als Verdicker können zusätzlich natürlich vorkommende Gelbildner eingesetzt werden, die bevorzugt ausgewählt sind aus Agar, Xanthan Gum, Cellulose und oder Cellulose-Derivaten oder Alginsäure.

Im Rahmen der Anmeldung sollen unter Pflegemitteln Substanzen verstanden werden, welche die Haare und/oder die Kopfhaut pflegen. Hydrolysiertes Weizenprotein, Panthenol und Allantoin wirken pflegend auf Kopfhaut und Haare. Hydrolysiertes Weizenprotein hat hauptsächlich feuchtigkeitsspendende Eigenschaften.

Als weitere Farbstoffe können in der erfindungsgemäßen Formulierung Anfärbestoffe enthalten sein, beispielsweise um der Formulierung eine optisch ansprechende Farbe zu geben. In der erfindungsgemäßen Formulierung kann z.B. CI 47005 als geeigneter Anfärbestoff enthalten sein.

Als Lösungsmittel kann ein für den Fachmann auf diesem Gebiet gängiges Lösungsmittel oder Lösungsmittelgemisch eingesetzt werden. Bevorzugte Lösungsmittel sind Ethanol oder Butylenglykol, insbesondere 1,4-Butylenglykol, Propylenglykol und Isopropylalkohol. Bevorzugt ist Ethanol bzw. Propylenglykol. Diese Lösungsmittel können vorzugsweise in einer Menge von 0,1 bis 70 Gew.% in der erfindungsgemäßen Formulierung enthalten sein. Ganz besonders bevorzugt sind sie in einer Menge von 0,1 bis 5,0 Gew.% enthalten.

Festigende Verbindungen sind vorzugsweise aus der Gruppe bestehend aus Wachsen, synthetischen Polymeren und Mischungen davon ausgewählt. Geeignete festigende Verbindungen sind dem Fachmann bekannt und beispielsweise in Goddard, E. Desmond; Gruber, James V. (1999), "Principles of polymer science and technology in cosmetics and personal care", Series: Cosmetic science and technology series : v. 22., New York (Marcel Dekker, INC.); Schrader, Karlheinz (1989), "Grundlagen und Rezepturen der Kosmetika" (2. Auflage), Kapitel 3.7, Heidelberg (Hüthig); sowie Scott, Richard (2017), "ingredients focus:cosmetic waxes and butters", Personal Care Europe 10(3), 46-47 beschrieben.

Antischuppenwirkstoffe werden eingesetzt, um übermäßige Bildung von Schuppen auf der Kopfhaut zu kontrollieren. Geeignete Antischuppenwirkstoffe sind dem Fachmann bekannt und beispielsweise in Trüeb, R. M. (2009). "Kopfschuppen erfolgreich behandeln." Akt Dermatol 35(01/02): 19-24; Trueb, R. M. (2007). "Shampoos: ingredients, efficacy and adverse effects." J. Dtsch. Dermatol. Ges. 5(5): 356-365; Sanfilippo, A. and J. English (2006). "An overview of medicated shampoos used in dandruff treatment." P AND T 31(7): 396; sowie Futterer, E. (1981). "Evaluation of efficacy of antidandruff agents." J Soc Cosmet Chem 32: 327-338 beschrieben.

Vitamine und Mineralstoffe wie Niacinamid (Vitamin B3), Vitamin E oder Zink werden eingesetzt, um einen positiven Effekt auf das Wachstum und die Reparatur von Haarschädigungen zu erzielen. Geeignete Vitamine und Mineralstoffe sind dem Fachmann bekannt und beispielsweise in Pietrzik K, Golly I, Loew D, Handbuch Vitamine, Elsevier GmbH, Urban & Fischer Verlag, München 2008; H. Lautenschläger, Vitamine in der Kosmetik, medical Beauty Forum 2020 (3), 14-17; sowie Martini M.C., Chivot M., Peyrefitte G., Lehrbuch Kosmetik: Grundlagen - Grundstoffe - Grundtechniken, Hogrefe AG, Bern 2001 beschrieben.

Alkalisierungsmittel bzw. pH-Stellmittel werden eingesetzt, um den pH-Wert der Formulierung auf einen gewünschten Bereich einzustellen. Geeignete Alkalisierungsmittel bzw. pH-Stellmittel umfassen unter anderem Natriumhydroxid, Monoethanolamin 0,1-5%, Phosphorsäure und Citronensäure.

Beispielhafte Zusammensetzungen sind in der folgenden Tabelle dargestellt:

| Bestandteil | Menge in Gew.-%% |
|---|---|
| Aqua [Wasser] | ad 100 |
| Natriumlaurylethersulfat | 5-15 |
| Natriummyrethsulfat | 0-5 |
| Natrium-Cocoamphoacetat | 0-5 |
| Laureth-2 | 0-5 |
| Cocoamidopropylbetain | 0-5 |
| Dinatriumlaurylsulfosuccinat | 0-5 |
| Natriumlauroylglutamat | 0-5 |
| Panthenol | 0-5 |
| Koffein | 0,1-5 |
| Natriumchlorid | 0-5 |
| Phenoxyethanol | 0-5 |
| Weizenproteinhydrolysat | 0-3 |
| Cocoglucosid | 0-3 |
| Glyceryloleat | 0-3 |
| Phosphorsäure | 0-3 |
| Natriumbenzoat | 0-3 |
| PEG-120 Methylglucosedioleat | 0-3 |
| Kaliumsorbat | 0-3 |
| HC Blue 2 | 0,1-2 |
| Allantoin | 0-3 |
| PEG-40 hydriertes Rizinusöl | 0-3 |
| Menthol | 0-2 |
| Polyquaternium-7 | 0-2 |
| Natriumhydroxid | 0-2 |
| Dinatrium-EDTA | 0-2 |
| Dihydroxyindol | 0,01-2 |
| Tocopherol | 0-2 |
| Zitronensäure | 0-2 |
| Niacinamid | 0-2 |
| Zink PCA | 0-2 |
| Farbstoff / Anfärbestoff | 0-2 |

Erfindungsgemäß wird die Zusammensetzung topisch angewendet. Unter einer topischen Anwendung ist eine äußerliche Anwendung, insbesondere eine örtliche, äußerliche Anwendung zu verstehen. Erfindungsgemäße Zusammensetzungen sind bevorzugt Haarpflegeprodukte (d.h. Behandlungsmittel wie Shampoos, Conditioner oder Kuren).

In einer bevorzugten Ausführungsform liegt die erfindungsgemäße Formulierung als Shampoo, Conditioner, Kur oder Farbkur vor, insbesondere als Shampoo oder Farbkur.

Die kosmetische Formulierung der vorliegenden Erfindung ist insbesondere zur Pigmentierung und/oder Repigmentierung von Keratinfasern geeignet.

Dementsprechend betrifft die vorliegende Erfindung in einer weiteren Ausführungsform die Verwendung der kosmetischen Formulierung enthaltend ein Farbstoffvorprodukt, ausgewählt aus der Gruppe bestehend aus den in Anspruch 1 definierten Indolin-Derivaten und Indol-Derivaten, den Xanthin-Derivat Koffein und mindestens einen direktziehenden Farbstoff, als Bioaktivstoff zur Pigmentierung und/oder Repigmentierung von Keratinfasern.

Unter "Keratinfasern" sind im Sinne der vorliegenden Erfindung Haare zu verstehen, insbesondere menschliche Haare. Der Begriff "menschliche Haare" umfasst hierbei insbesondere Kopfhaare und Barthaare.

In einer bevorzugten Ausführungsform betrifft die vorliegende Erfindung die Verwendung der kosmetischen Formulierung zur Pigmentierung und/oder Repigmentierung von menschlichen Haaren.

Hierbei wird die Formulierung vorzugsweise im nassen oder trockenen Haar verteilt und nach einer Verweildauer zwischen 1 und 30 Minuten wieder ausgespült.

Die erfindungsgemäße kosmetischen Formulierung zur Pigmentierung und/oder Repigmentierung von Keratinfasern, insbesondere menschlichen Haaren, bewirkt, dass die Haare langsam und schonend permanent gefärbt und gleichzeitig die Keratinozyten in den Haarwurzeln angeregt werden, damit diese wieder verstärkt natürliches Pigment produzieren.

Somit betrifft die Erfindung in einer weiteren Ausführungsform die Verwendung der kosmetischen Formulierung zur Pigmentierung und/oder Repigmentierung von menschlichen Haaren, wobei die Haare langsam und schonend permanent gefärbt und gleichzeitig die Keratinozyten in den Haarwurzeln angeregt werden, damit diese wieder verstärkt natürliches Pigment produzieren.

In einer weiteren Ausführungsform betrifft die Erfindung ein Verfahren zur Pigmentierung und/oder Repigmentierung von Keratinfasern, insbesondere menschlichen Haaren, wobei die erfindungsgemäße kosmetische Formulierung auf das Haar aufgebracht wird und anschließend die Ausfärbung erfolgt. Die Ausfärbung wird lediglich durch Luftsauerstoff bewirkt, es ist kein weiteres Oxidationsmittel erforderlich.

Anhand der nachfolgenden Formulierungen soll die Erfindung verdeutlicht werden, ohne sie jedoch auf die speziellen Beispiele einschränken zu wollen.

### Figurenbeschreibung

**Figur 1** Vergleichsfotos von mit Beispielrezeptur 2 (D1/D2) bzw. Vergleichsrezeptur (E1/E2) gewaschenen Strähnen
**Figur 2** Vergleich der Farbvorschau für die beiden verwendeten Shampoos Beispielrezeptur 2 bzw. Vergleichsrezeptur
**Figur 3** Veränderung der Farbtiefe bei Verwendung der Shampoos Beispielrezeptur 2 bzw. Vergleichsrezeptur als Absolutwerte dL*
**Figur 4** Farbveränderung gesamt dE* bei Verwendung der Shampoos Beispielrezeptur 2 bzw. Vergleichsrezeptur
**Figur 5** Veränderung der Farbtiefe beim Aufwaschen mit Färbeshampoo (5 Waschgänge) bzw. Auswaschen mit Neutralrezeptur (14 Waschgänge)
**Figur 6** Veränderung der Farbtiefe beim Aufwaschen mit Färbeshampoo (10 Waschgänge) bzw. Auswaschen mit Neutralrezeptur (15 Waschgänge)
**Figur 7** Veränderung der Farbtiefe beim Aufwaschen mit Färbeshampoo (15 Waschgänge) bzw. Auswaschen mit Neutralrezeptur (15 Waschgänge)
**Figur 8** Vergleich der Farbveränderung für die beiden verwendeten Shampoos auf jeweils einer einzelnen Strähne bei 15 Aufwaschgängen, gefolgt von 15 Auswaschgängen
**Figur 9** Vergleich der Farbvorschau für die verwendeten Shampoos Beispielrezeptur 1 bzw. Beispielrezeptur 2 bzw. Vergleichsrezeptur auf jeweils einer einzelnen Strähne bei 15 Aufwaschgängen
**Figure 10** Veränderung der Farbtiefe bei Verwendung der Shampoos Beispielrezeptur 1 bzw. Beispielrezeptur 2 bzw. Vergleichsrezeptur als Absolutwerte dL*
**Figur 11** Farbveränderung gesamt dE* bei Verwendung der Shampoos Beispielrezeptur 1 bzw. Beispielrezeptur 2 bzw. Vergleichsrezeptur

### Experimenteller Teil

### Rezepturen

Die folgenden Rezepturen wurden durch für den Fachmann bekannte Maßnahmen hergestellt.

### Vergleichsrezeptur

Die Vergleichsrezeptur enthält Koffein als Xanthin-Derivat und HC Blue 2 als direktziehenden Farbstoff.

| Bestandteil | Menge in Gew.-% |
|---|---|
| Aqua [Wasser] | ad 100 |
| Natriumlaurylethersulfat | 11,5 |
| Natrium-Cocoamphoacetat | 3 |
| Cocamidopropylbetain | 1,7 |
| Koffein | 1,1 |
| Panthenol | 0,92 |
| Natriumchlorid | 1 |
| Phenoxyethanol | 0,7 |
| Cocoglucosid | 0,5 |
| Glyceryloleat | 0,5 |
| Phosphorsäure | 0,4 |
| PEG-120 Methylglucosedioleat | 0,3 |
| HC Blue 2 | 0,22 |
| Menthol | 0,2 |
| Polyquaternium-7 | 0,2 |
| Natriumhydroxid | 0,15 |
| Niacinamid | 0,1 |
| Zink PCA | 0,1 |
| Farbstoff / Anfärbestoff | 0,1 |
| Zitronensäure | 0,06 |

### Beispielrezeptur 1 (außerhalb der Erfindung)

Die Beispielrezeptur 1 enthält Dihydroxyindol und Koffein als Xanthin-Derivat.

| Bestandteil | Menge in Gew.-% |
|---|---|
| Aqua [Wasser] | ad 100 |
| Natriumlaurylethersulfat | 8,0 |
| Natriummyrethsulfat | 2,5 |
| Laureth-2 | 2 |
| Panthenol | 1,7 |
| Natriumchlorid | 1,65 |
| Dinatriumlaurylsulfosuccinat | 1,5 |
| Natriumlauroylglutamat | 1,3 |
| Koffein | 1,1 |
| PEG-120 Methylglucosedioleat | 0,75 |
| Weizenproteinhydrolysat | 0,7 |
| Dihydroxyindol | 0,49 |
| Allantoin | 0,4 |
| Zitronensäure | 0,38 |
| Natriumbenzoat | 0,36 |
| Propylenglykol | 0,3 |
| Kaliumsorbat | 0,3 |
| PEG-40 hydriertes Rizinusöl | 0,18 |
| Cocoglucosid | 0,18 |
| Glyceryloleat | 0,17 |
| Polyquaternium-7 | 0,17 |
| Dinatrium-EDTA | 0,15 |
| Zink PCA | 0,1 |
| Niacinamid | 0,1 |
| Tocopherol | 0,06 |

### Beispielrezeptur 2

Die Beispielrezeptur 2 enthält Dihydroxyindol, Koffein als Xanthin-Derivat und HC Blue 2 als direktziehenden Farbstoff.

| Bestandteil | Menge in Gew.-% |
|---|---|
| Aqua [Wasser] | ad 100 |
| Natriumlaurylethersulfat | 11,5 |
| Natrium-Cocoamphoacetat | 3 |
| Cocamidopropylbetain | 1,7 |
| Koffein | 1,1 |
| Panthenol | 0,92 |
| Natriumchlorid | 1 |
| Phenoxyethanol | 0,7 |
| Cocoglucosid | 0,5 |
| Glyceryloleat | 0,5 |
| Phosphorsäure | 0,4 |
| PEG-120 Methylglucosedioleat | 0,3 |
| HC Blue 2 | 0,22 |
| Menthol | 0,2 |
| Polyquaternium-7 | 0,2 |
| Natriumhydroxid | 0,15 |
| Dihydroxyindol | 0,2 |
| Niacinamid | 0,1 |
| Zink PCA | 0,1 |
| Farbstoff / Anfärbestoff | 0,1 |
| Zitronensäure | 0,06 |

### Beispielrezeptur 3

Die Beispielrezeptur 3 enthält Dihydroxyindol, Koffein als Xanthin-Derivat und HC Blue 2 als direktziehenden Farbstoff.

| Bestandteil | Menge in Gew.-% |
|---|---|
| Aqua [Wasser] | ad 100 |
| Natriumlaurylethersulfat | 9 |
| Natriummyrethsulfat | 3 |
| Natrium-Cocoamphoacetat | 1 |
| Laureth-2 | 2 |
| Dinatriumlaurylsulfosuccinat | 1 |
| Natriumlauroylglutamat | 2 |
| Koffein | 0,9 |
| Natriumchlorid | 1 |
| Weizenproteinhydrolysat | 0,7 |
| Phosphorsäure | 0,3 |
| Natriumbenzoat | 0,5 |
| Kaliumsorbat | 0,2 |
| HC Blue 2 | 1 |
| Allantoin | 0,4 |
| PEG-40 hydriertes Rizinusöl | 0,4 |
| Polyquaternium-7 | 0,2 |
| Dinatrium-EDTA | 0,2 |
| Dihydroxyindol | 0,3 |
| Tocopherol | 0,02 |

### Beispielrezeptur 4

Die Beispielrezeptur 4 enthält Dihydroxyindol, Koffein als Xanthin-Derivat und HC Blue 2 als direktziehenden Farbstoff.

| Bestandteil | Menge in Gew.-% |
|---|---|
| Aqua [Wasser] | ad 100 |
| Natriumlaurylethersulfat | 11 |
| Natriummyrethsulfat | 2 |
| Laureth-2 | 3 |
| Cocoamidopropylbetain | 2 |
| Dinatriumlaurylsulfosuccinat | 1 |
| Natriumlauroylglutamat | 1 |
| Panthenol | 0,5 |
| Koffein | 1 |
| Natriumchlorid | 0,9 |
| Phenoxyethanol | 0,5 |
| Weizenproteinhydrolysat | 0,5 |
| Cocoglucosid | 0,3 |
| Glyceryloleat | 0,4 |
| Phosphorsäure | 0,25 |
| Natriumbenzoat | 0,2 |
| PEG-120 Methylglucosedioleat | 0,1 |
| Kaliumsorbat | 0,5 |
| HC Blue 2 | 0,8 |
| Allantoin | 0,1 |
| PEG-40 hydriertes Rizinusöl | 0,2 |
| Menthol | 0,2 |
| Natriumhydroxid | 0,1 |
| Dinatrium EDTA | 0,2 |
| Dihydroxyindol | 0,1 |
| Zitronensäure | 0,03 |
| Niacinamid | 0,2 |
| Zink PCA | 0,2 |
| Farbstoff / Anfärbestoff | 0,1 |

### Beispielrezeptur 5 (außerhalb der Erfindung)

Die Beispielrezeptur 5 enthält Dihydroxyindol und Koffein als Xanthin-Derivat.

| Bestandteil | Menge in Gew.-% |
|---|---|
| Aqua [Wasser] | ad 100 |
| Natriumlaurylethersulfat | 12 |
| Natrium-Cocoamphoacetat | 2 |
| Cocoamidopropylbetain | 2 |
| Panthenol | 1 |
| Koffein | 1,1 |
| Natriumchlorid | 0,9 |
| Phenoxyethanol | 0,8 |
| Cocoglucosid | 0,4 |
| Glyceryloleat | 0,3 |
| Phosphorsäure | 0,3 |
| PEG-120 Methylglucosedioleat | 0,2 |
| Menthol | 0,2 |
| Polyquaternium-7 | 0,1 |
| Natriumhydroxid | 0,2 |
| Dinatrium EDTA | 0,1 |
| Dihydroxyindol | 0,2 |
| Zitronensäure | 0,06 |
| Niacinamid | 0,2 |
| Zink PCA | 0,2 |
| Farbstoff / Anfärbestoff | 0,5 |

### Neutralrezeptur

Die Neutralrezeptur enthält **weder** Koffein **noch** DHI **noch** HC Blue 2.

| Bestandteil | Menge in Gew.-% |
|---|---|
| Aqua [Wasser] | ad 100 |
| Natriumlaurylethersulfat | 10 |
| Laureth-2 | 1,9 |
| Dinatriumlaurylsulfosuccinat | 1,5 |
| Natriumchlorid | 1,3 |
| Natriumlauroylglutamat | 1,3 |
| Panthenol | 1,2 |
| PEG-120 Methylglucosedioleat | 0,5 |
| Weizenproteinhydrolysat | 0,49 |
| Zitronensäure | 0,4 |
| Natriumcitrat | 0,35 |
| Propylenglykol | 0,2 |
| Menthol | 0,2 |
| PEG-40 hydriertes Rizinusöl | 0,2 |
| Kaliumsorbat | 0,19 |
| Polyquaternium-7 | 0,17 |
| Dinatrium EDTA | 0,11 |
| Natriumbenzoat | 0,1 |
| Zink PCA | 0,06 |
| Niacinamid | 0,055 |
| Tocopherol | 0,032 |
| Farbstoff / Anfärbestoff | 0,0005 |

### Anwendungsbeispiele

Zur Bewertung der Färbeleistung hinsichtlich Qualität, Intensität und Dauer wurden die folgenden Waschtests (Aufwasch- sowie Auswaschversuche) durchgeführt.

### Anwendungsbeispiel 1

Kerling-Strähnen wurden einem Aufwaschversuch mit einem Shampoo enthaltend 5,6-DHI, Koffein und HC Blue 2 (Beispielrezeptur 2) bzw. mit einem Shampoo enthaltend HC Blue 2 und Koffein (Vergleichsrezeptur) unterzogen, um die Färbeleistung hinsichtlich Qualität und Intensität zu untersuchen.

### Waschprotokoll:

- Strähnen 30 s mit lauwarmem Wasser befeuchten, überschüssiges Wasser zwischen zwei Fingern ausstreichen
- Pro 5 Strähnen insgesamt 5 g Shampoo 1 min einmassieren
- Insgesamt 5 Minuten einwirken lassen
- 1 Minute mit lauwarmem Wasser ausspülen, anschließend in Küchenrolle ausdrücken
- Kämmen, einzeln ausgebreitet an der Luft trocknen
- Am nächsten Tag Strähnen mit dem Konica (Konica-Minolta Spectrophotometer CM600d, Messgeometrie d/8°, Specular Component Excluded (SCE), Normlichtart D65, Gesichtsfeld 10°) einlesen (alle 5 Waschgänge Beispielsträhnen fotografieren); dann Waschzyklus wiederholen

### Verwendete Haarsträhnen:

Kerling weiß selektiert, Klebetresse 2 cm breit, 12 cm lang, 3 g/St., Ch. 1719

### Verwendete Shampoos:

| | |
|---|---|
| Beispielrezeptur 2 | Strähnen D1-D5 |
| Vergleichsrezeptur | Strähnen E1-E5 |

Pro Produkt wurden jeweils 5 Strähnen verwendet, es wurden jeweils 28 Waschgängen durchgeführt.

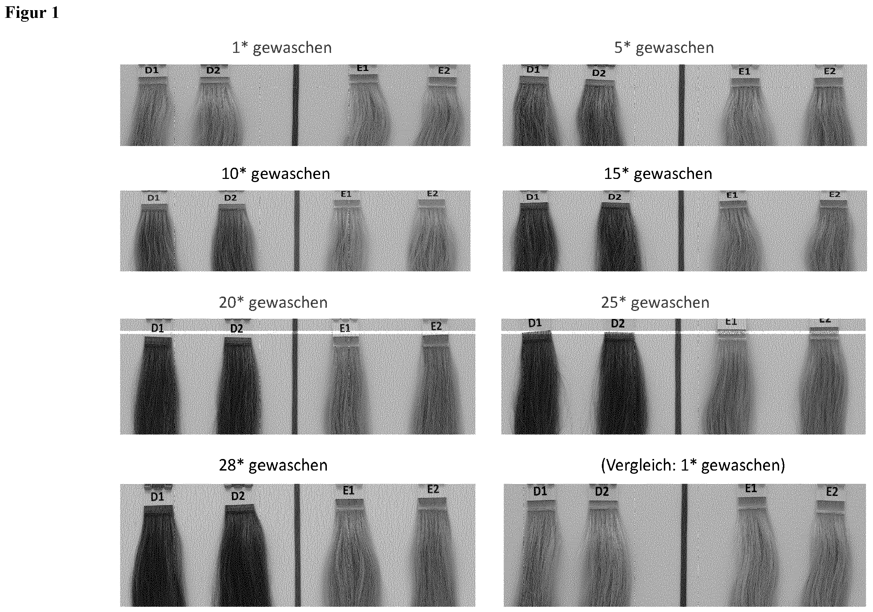

Figur 1 zeigt einen Vergleich der mit Beispielrezeptur 2 behandelten Strähnen D1/D2 mit Strähnen E1/E2, welche mit der Vergleichsrezeptur behandelt wurden.

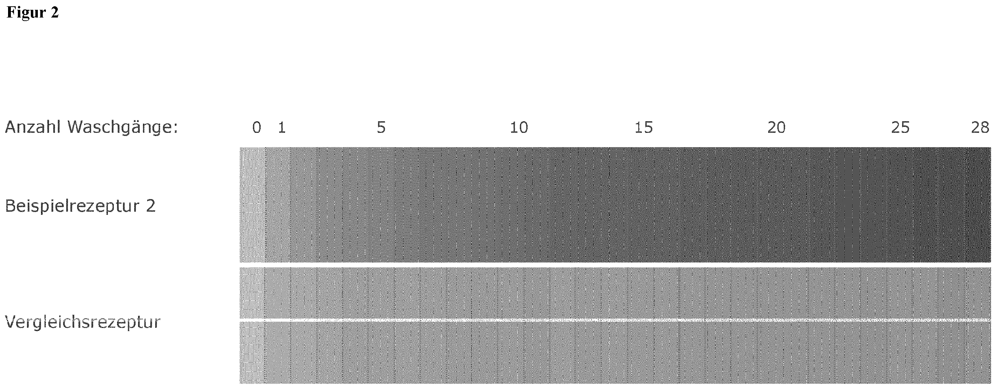

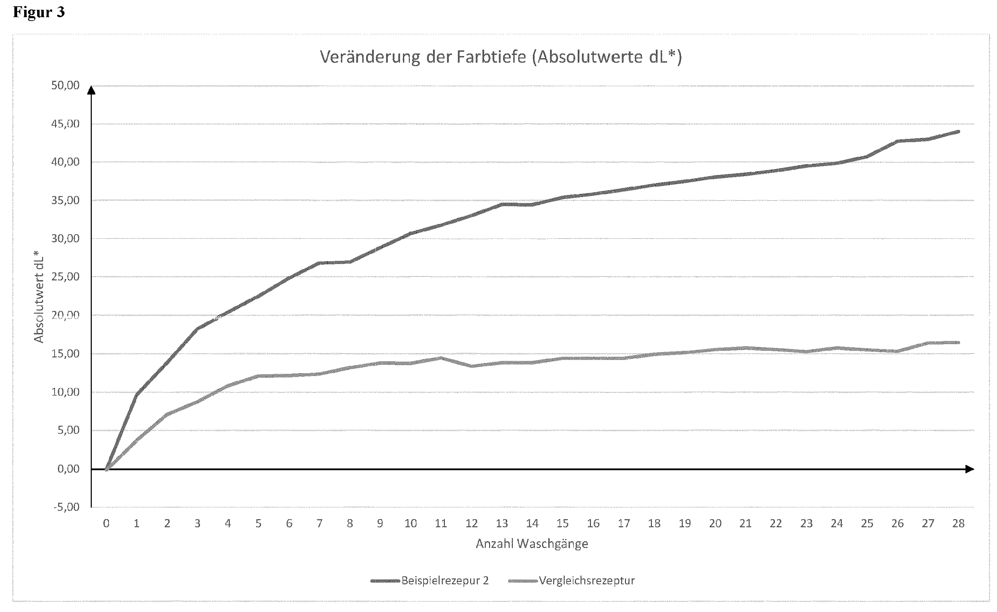

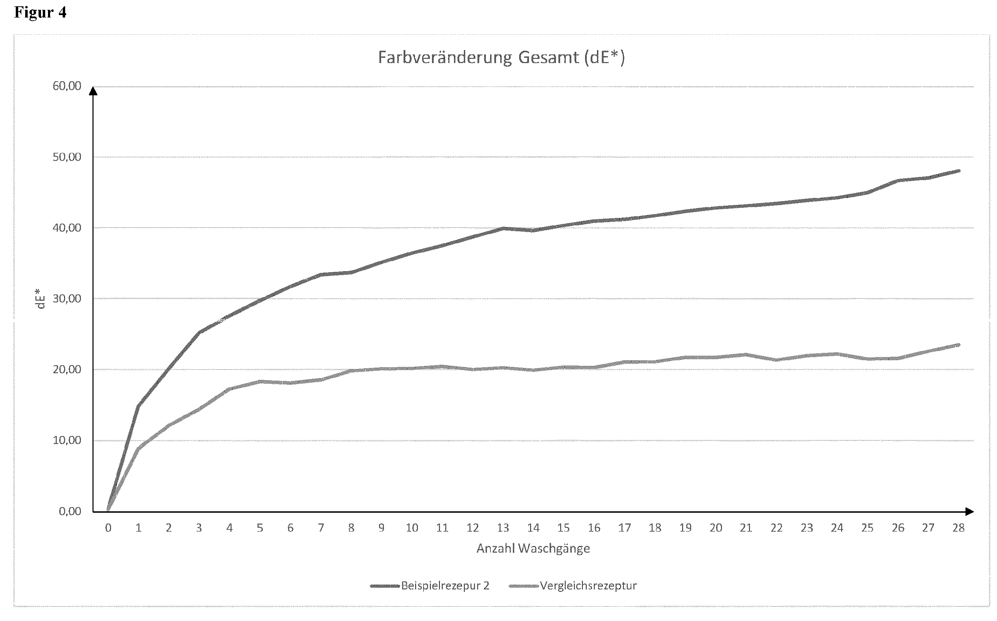

Der Vergleich der Farbvorschau für die beiden verwendeten Shampoos ist in Figur 2 dargestellt. Die Figuren 3 und 4 zeigen die Veränderung der Farbtiefe als Absolutwerte dL* (Figur 3) bzw. als Farbveränderung gesamt dE* (Figur 4). Aus den Figuren ist der graduelle Aufbau des Farbeffekts bei wiederholter Anwendung ersichtlich sowie das gegenüber der Vergleichsrezeptur verbesserte Färbergebnis.

### Anwendungsbeispiel 2

Kerling-Strähnen wurden einem Aufwasch- und Auswaschversuch mit einem Shampoo enthaltend 5,6-DHI, Koffein und HC Blue 2 (Beispielrezeptur 2) bzw. mit einem Shampoo enthaltend HC Blue 2 und Koffein (Vergleichsrezeptur) unterzogen, um die Färbeleistung hinsichtlich Qualität, Intensität und Dauer zu untersuchen.

### Waschprotokoll:

- Strähnen 30 s mit lauwarmem Wasser befeuchten, überschüssiges Wasser zwischen zwei Fingern ausstreichen
- Pro 5 Strähnen insgesamt 5 g Shampoo 1 min einmassieren
- Insgesamt 5 Minuten einwirken lassen
- 1 Minute mit lauwarmem Wasser ausspülen, anschließend in Küchenrolle ausdrücken
- Kämmen, einzeln ausgebreitet an der Luft trocknen
- Am nächsten Tag Strähnen mit dem Konica (Konica-Minolta Spectrophotometer CM600d, Messgeometrie d/8°, Specular Component Excluded (SCE), Normlichtart D65, Gesichtsfeld 10°) einlesen, fotografieren; dann Waschzyklus wiederholen

### Verwendete Haarsträhnen:

Kerling weiß selektiert, Klebetresse 2cm breit, 12 cm lang

### Verwendete Färbeshampoos:

| | |
|---|---|
| Beispielrezeptur 2 | Strähnen B1-B15 |
| Vergleichsrezeptur | Strähnen C1-C15 |

Zum Auswaschen wurde die Neutralrezeptur-verwendet.

Pro Produkt wurden jeweils 15 Strähnen in Portionen à 5 Stück behandelt.

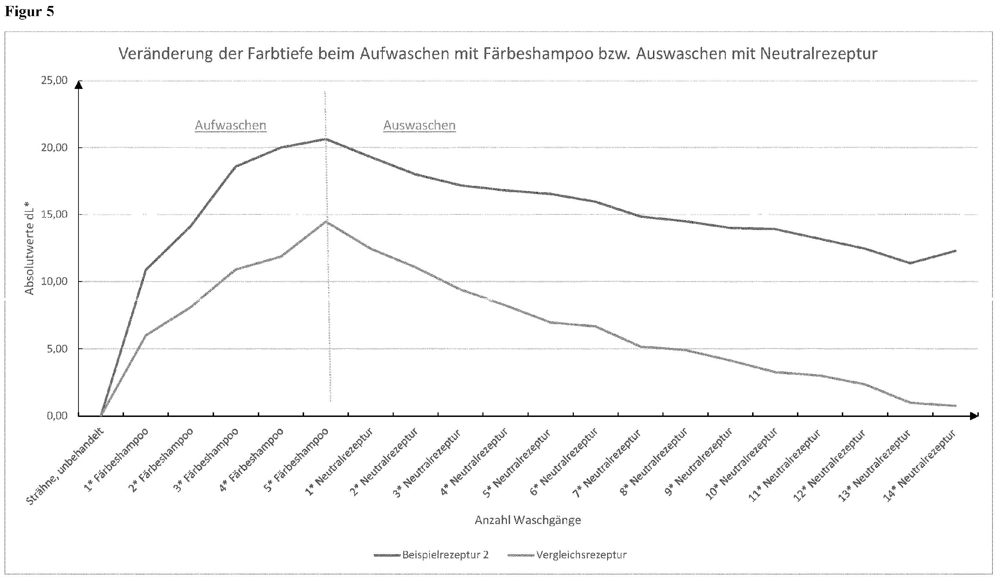

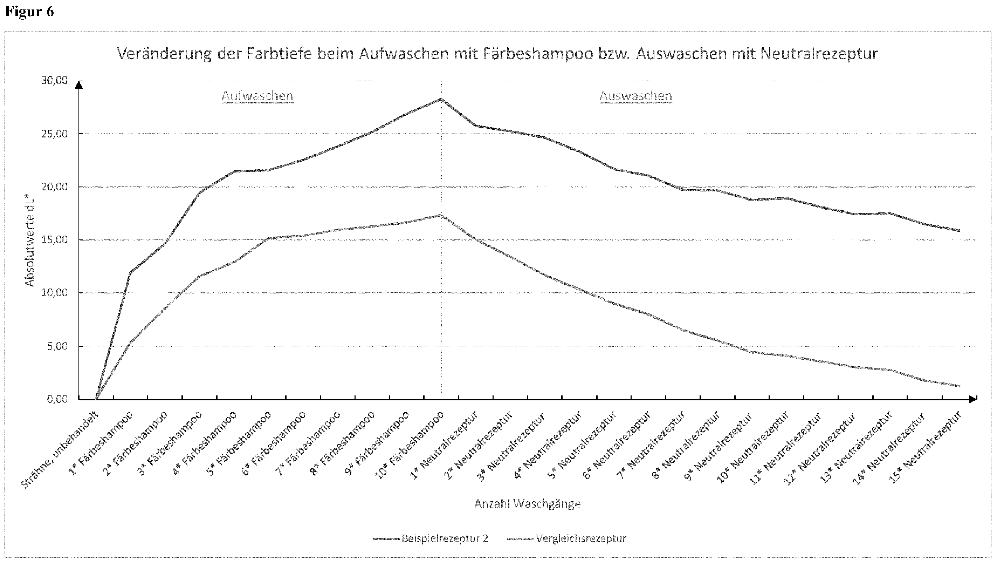

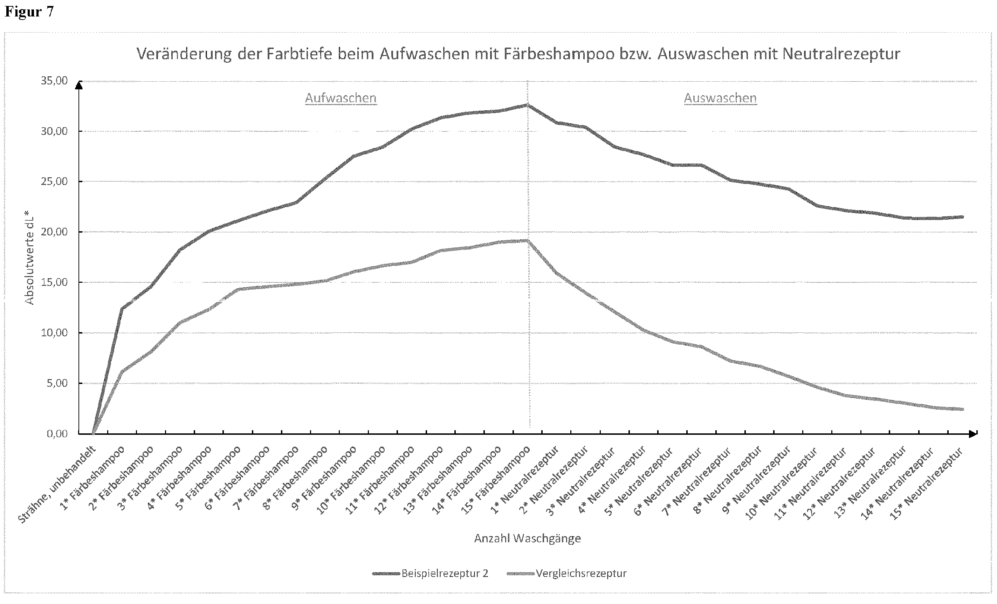

Die Farbveränderung wurde über die folgenden Aufwasch- und Auswaschgänge verfolgt:
- jeweils 5 Waschgänge mit Färbeshampoo zum Aufwaschen, gefolgt von 14 Waschgängen mit Neutralrezeptur (Figur 5)
- jeweils 10 Waschgänge mit Färbeshampoo zum Aufwaschen, gefolgt von 15 Waschgängen mit Neutralrezeptur (Figur 6)
- jeweils 15 Waschgänge mit Färbeshampoo zum Aufwaschen, gefolgt von 15 Waschgängen mit Neutralrezeptur (Figur 7).

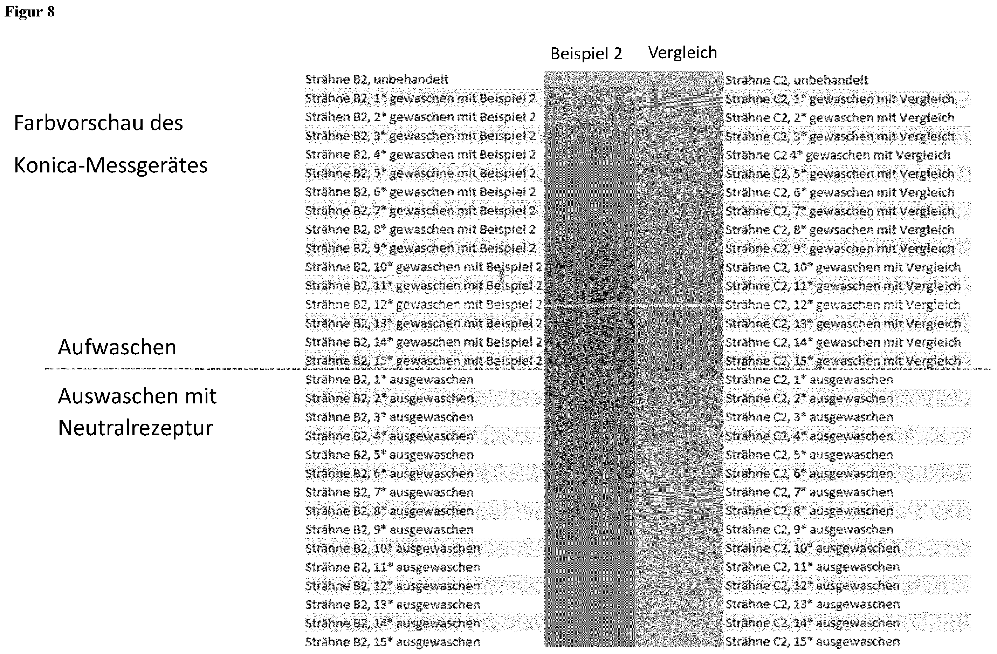

Die Figuren 5-7 zeigen jeweils die Veränderung der Farbtiefe beim Aufwaschen mit Färbeshampoo bzw. Auswaschen mit Neutralrezeptur gemäß den oben angegebenen Protokollen. Figur 8 zeigt den Vergleich der Farbveränderung für die beiden verwendeten Shampoos auf jeweils einer einzelnen Strähne bei 15 Aufwaschgängen, gefolgt von 15 Auswaschgängen, gemessen mit dem Konica-Messgerät (Konica-Minolta Spectrophotometer CM600d, Messgeometrie d/8°, Specular Component Excluded (SCE), Normlichtart D65, Gesichtsfeld 10°). Aus den Figuren ist der graduelle Aufbau des Farbeffekts bei wiederholter Anwendung ersichtlich sowie das gegenüber der Vergleichsrezeptur verbesserte Färbergebnis. Nach Absetzen der Behandlung mit dem erfindungsgemäßen Shampoo hält der Farbeffekt zudem länger an als bei Verwendung der Vergleichsrezeptur.

### Anwendungsbeispiel 3

White Virgin-Strähnen (Fa. Imhair) wurden einem Aufwaschversuch mit einem Shampoo enthaltend 5,6-DHI und Koffein (Beispielrezeptur 1) bzw. mit einem Shampoo enthaltend 5,6-DHI, Koffein und HC Blue 2 (Beispielrezeptur 2) bzw. mit einem Shampoo enthaltend HC Blue 2 und Koffein (Vergleichsrezeptur) unterzogen, um die Färbeleistung hinsichtlich Qualität und Intensität zu untersuchen.

### Waschprotokoll:

- Strähnen 30 s mit lauwarmem Wasser befeuchten, überschüssiges Wasser zwischen zwei Fingern ausstreichen
- Pro 3 Strähnen insgesamt 2 g Shampoo 30 s einmassieren
- Insgesamt 5 Minuten einwirken lassen
- 1 Minute mit lauwarmem Wasser ausspülen
- Kämmen, an der Luft trocknen
- Am nächsten Tag Strähnen mit dem Konica einlesen, fotografieren; dann Waschzyklus wiederholen

### Verwendete Haarsträhnen:

White Virgin (Fa. Imhair)

### Verwendete Shampoos:

| | |
|---|---|
| Beispielrezeptur 1 | Strähnen 1-3 |
| Beispielrezeptur 2 | Strähnen 4-6 |
| Vergleichsrezeptur | Strähnen 7-9 |

Pro Produkt wurden jeweils 3 Strähnen verwendet, es wurden jeweils 15 Waschgängen durchgeführt.

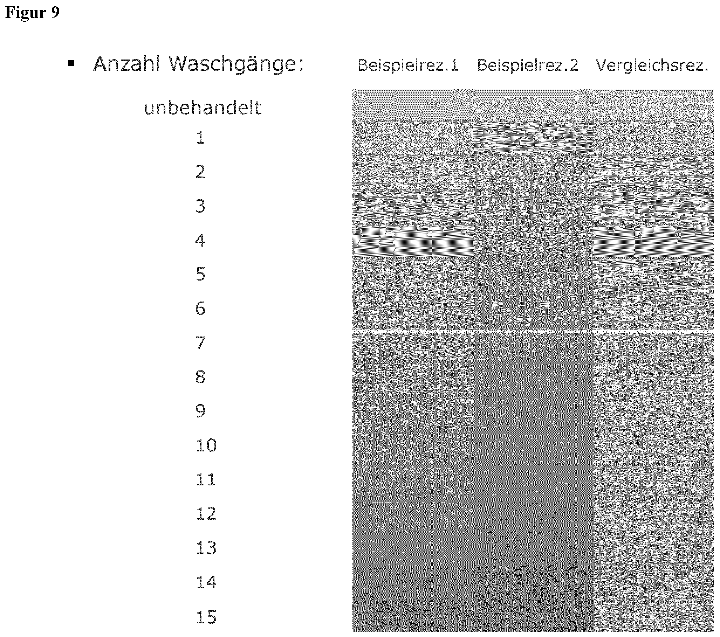

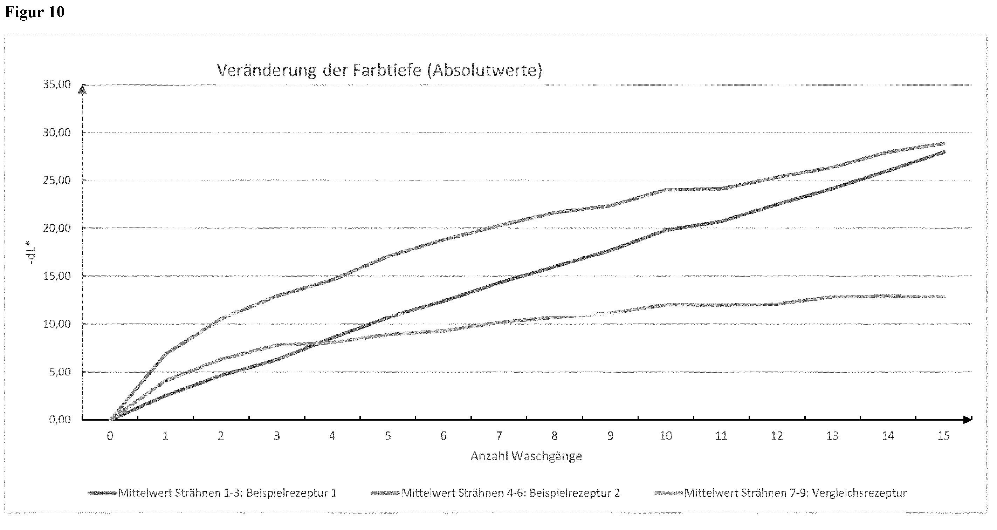

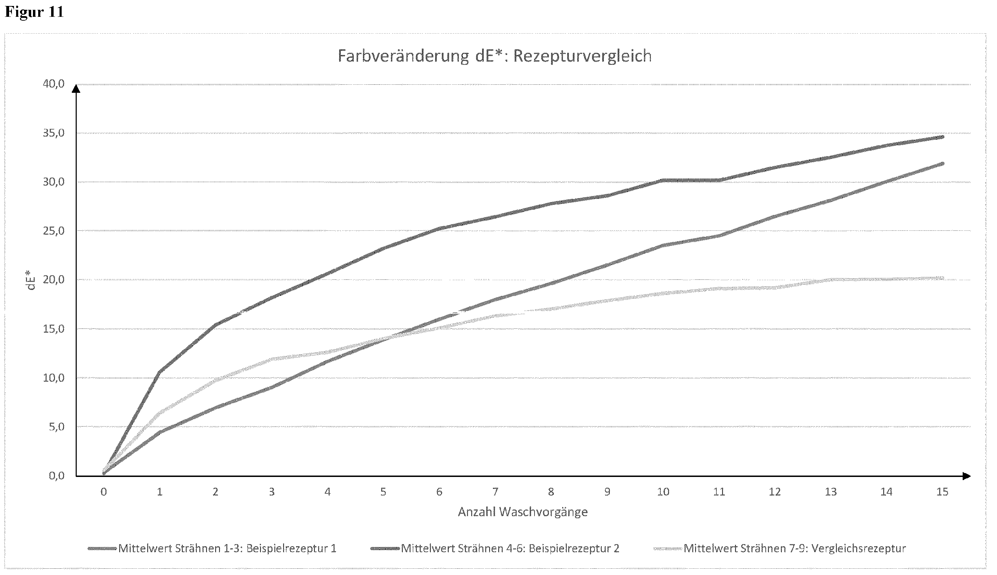

Der Vergleich der Farbvorschau für die verwendeten Shampoos ist jeweils für eine einzelne Haarsträhne in Figur 9 dargestellt. Die Figuren 10 und 11 zeigen die Veränderung der Farbtiefe als Absolutwerte dL* (Figur 10) bzw. als Farbveränderung gesamt dE* (Figur 11). Aus den Figuren ist der graduelle Aufbau des Farbeffekts bei wiederholter Anwendung ersichtlich sowie das gegenüber der Vergleichsrezeptur verbesserte Färbergebnis.

### Anwendungsbeispiel 4

Zur Untersuchung der ausgefärbten Nuance über die Zeit wurden die Strähnen Kerling white und Imhair blondiert 15 min mit 10 g Beispielrezeptur 2 gefärbt, 1 min ausgespült und 1,5 h an der Luft getrocknet (Restfeuchte wurde dann trockengeföhnt). Es wurden Messungen am Tag der Behandlung nach 15 min Einwirkzeit (Ausgangsnuance) sowie nach 1, 2, 3, 8, 9, 10, 11, 14 und 15 Tagen vorgenommen.

| | L*(D65) | a*(D65) | b*(D65) | dL*(D65) | da*(D65) | db*(D65) | dE*ab(D65) |
|---|---|---|---|---|---|---|---|
| **Strähne Kerling, white** | 76,58 | 2,15 | 21 | ------ | ------ | ------ | ------ |
| Ausgangsnuance (nach 15 min Einwirkzeit) | 62,32 | 1,58 | 5,02 | -14,27 | -0,57 | -15,98 | 21,43 |
| eingelesen nach 1d | 62,11 | 2,05 | 5,23 | -14,48 | -0,1 | -15,77 | 21,41 |
| eingelesen nach 2d | 63,06 | 2,22 | 4,59 | -13,52 | 0,07 | -16,42 | 21,27 |
| eingelesen nach 3d | 63,02 | 2,44 | 5,09 | -13,56 | 0,29 | -15,91 | 20,91 |
| eingelesen nach 8d | 62,95 | 2,53 | 5,57 | -13,64 | 0,38 | -15,44 | 20,6 |
| eingelesen nach 9d | 62,86 | 2,35 | 5,5 | -13,73 | 0,21 | -15,51 | 20,71 |
| eingelesen nach 10d | 62,63 | 2,57 | 5,87 | -13,95 | 0,42 | -15,13 | 20,58 |
| eingelesen nach 11d | 62,59 | 2,61 | 5,47 | -14 | 0,46 | -15,53 | 20,91 |
| eingelesen nach 14d | 63,16 | 3,1 | 6,34 | -13,43 | 0,95 | -14,67 | 19,91 |
| eingelesen nach 15d | 63,59 | 3,16 | 6,26 | -12,99 | 1,02 | -14,74 | 19,68 |

| | L*(D65) | a*(D65) | b*(D65) | dL*(D65) | da*(D65) | db*(D65) | dE*ab(D65) |
|---|---|---|---|---|---|---|---|
| **Strähne Imhair, blondiert** | 76,9 | 3,5 | 24,61 | ------ | ------ | ------ | ------ |
| Ausgangsnuance (nach 15 min Einwirkzeit) | 51,51 | 1,01 | 2,73 | -25,38 | -2,49 | -21,88 | 33,61 |
| eingelesen nach 1d | 51,71 | 1,6 | 2,8 | -25,19 | -1,9 | -21,82 | 33,38 |
| eingelesen nach 2d | 52,11 | 1,61 | 2,79 | -24,78 | -1,89 | -21,82 | 33,08 |
| eingelesen nach 3d | 51,57 | 2,06 | 3,23 | -25,33 | -1,43 | -21,38 | 33,17 |
| eingelesen nach 8d | 52,21 | 2,22 | 3,78 | -24,68 | -1,28 | -20,84 | 32,33 |
| eingelesen nach 9d | 51,98 | 2,13 | 3,84 | -24,92 | -1,36 | -20,78 | 32,47 |
| eingelesen nach 10d | 52 | 2,29 | 4,16 | -24,9 | -1,21 | -20,46 | 32,24 |
| eingelesen nach 11d | 52,11 | 2,29 | 4,16 | -24,79 | -1,21 | -20,46 | 32,16 |
| eingelesen nach 14d | 52,69 | 2,65 | 4,86 | -24,21 | -0,84 | -19,75 | 31,25 |
| eingelesen nach 15d | 53,23 | 2,68 | 5,01 | -23,67 | -0,82 | -19,61 | 30,74 |

Die Messwerte belegen eine - auch optisch wahrnehmbare - Nuancenverschiebung vom ursprünglichen Graphit hin zu einem natürlicheren, bräunlicheren Farbton.

## Patentansprüche

1. Kosmetische Formulierung enthaltend ein Farbstoffvorprodukt, ausgewählt aus der Gruppe bestehend aus Derivaten des 5,6-Dihydroxyindols der Formel (1), 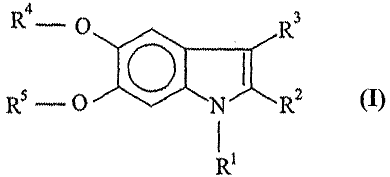 wobei, jeweils unabhängig voneinander,
R¹ Wasserstoff, eine C₁-C₄-Alkylgruppe oder eine C₁-C₄-Hydroxy-alkylgruppe bedeutet,
R² Wasserstoff oder eine-COOH-Gruppe bedeutet, wobei die-COOH-Gruppe auch als Salz mit einem physiologisch verträglichen Kation vorliegen kann,
R³ Wasserstoff oder eine C₁-C₄-Alkylgruppe bedeutet,
R⁴ Wasserstoff, eine C₁-C₄-Alkylgruppe, eine Aminogruppe oder eine Gruppe -CO-R⁶ bedeutet, in der R⁶ eine C₁-C₄-Alkylgruppe bedeutet, und
R⁵ eine der unter R⁴ genannten Gruppen bedeutet,
oder ein physiologisch verträgliches Salz dieser Verbindungen mit einer organischen oder anorganischen Säure, und
Derivaten des 5,6-Dihydroxyindolins der Formel (II), 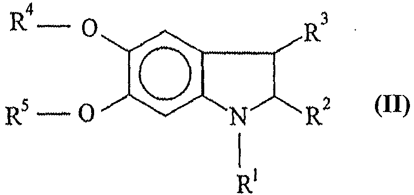 wobei, jeweils unabhängig voneinander,
R¹ Wasserstoff, eine C₁-C₄-Alkylgruppe oder eine C₁-C₄-Hydroxy-alkylgruppe bedeutet,
R² Wasserstoff oder eine-COOH-Gruppe bedeutet, wobei die-COOH-Gruppe auch als Salz mit einem physiologisch verträglichen Kation vorliegen kann,
R³ Wasserstoff oder eine C₁-C₄-Alkylgruppe bedeutet,
R⁴ Wasserstoff, eine C₁-C₄-Alkylgruppe, eine Aminogruppe oder eine Gruppe -CO-R⁶ bedeutet, in der R⁶ eine C₁-C₄-Alkylgruppe bedeutet, und
R⁵ eine der unter R⁴ genannten Gruppen bedeutet,
oder ein physiologisch verträgliches Salz dieser Verbindungen mit einer organischen oder anorganischen Säure;
ein Xanthin-Derivat als Bioaktivstoff, wobei das Xanthin-Derivat Koffein ist; und mindestens einen direktziehenden Farbstoff.

2. Kosmetische Formulierung nach Anspruch 1, wobei die Verbindung der Formel (I) ausgewählt ist aus 5,6-Dihydroxyindol, N-Methyl-5,6-dihydroxyindol sowie deren physiologisch verträglichen Salzen.

3. Kosmetische Formulierung nach Anspruch 1, wobei die Verbindung der Formel (II) ausgewählt ist aus 5,6-Dihydroxyindolin, N-Methyl-5,6-dihydroxyindolin sowie deren physiologisch verträglichen Salzen.

4. Kosmetische Formulierung nach einem der Ansprüche 1-3, wobei der direktziehende Farbstoff ausgewählt ist aus der Gruppe bestehend aus 2-Amino-6-chlor-4-nitrophenol, 1,4-Bis-(β-hydroxyethyl)-amino-2-nitrobenzol, Basic Yellow 57, Basic Red 76, Basic Brown 16, Basic Brown17, Basic Blue 99, HC Blue 2, HC Blue 12, HC Blue 16 und Acid Violet 43, bevorzugt HC Blue 2 ist.

5. Nicht-therapeutische Verwendung der kosmetischen Formulierung nach einem der vorhergehenden Ansprüche zur Repigmentierung von Keratinfasern, insbesondere menschlichen Haaren.

6. Verwendung nach Anspruch 5 zur Repigmentierung von menschlichen Haaren, wobei die Formulierung im nassen oder trockenen Haar verteilt wird und nach einer Verweildauer zwischen 1 und 30 Minuten wieder ausgespült wird.

7. Verwendung nach Anspruch 5 oder Anspruch 6 zur Repigmentierung von menschlichen Haaren, wobei die Haare langsam und schonend permanent gefärbt und gleichzeitig die Keratinozyten in den Haarwurzeln angeregt werden, damit diese wieder verstärkt natürliches Pigment produzieren.

8. Nicht-therapeutisches Verfahren zur Repigmentierung von Keratinfasern, insbesondere menschlichen Haaren, wobei die kosmetische Formulierung nach einem der Ansprüche 1-4 auf das Haar aufgebracht wird und anschließend die Ausfärbung erfolgt.

## Claims

1. Cosmetic formulation containing a dye precursor, selected from the group consisting of derivatives of 5,6-dihydroxyindole of formula (I), 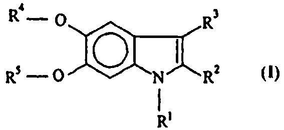 wherein, independently of one another,
R¹ means hydrogen, a C₁-C₄ alkyl group or a C₁-C₄ hydroxyalkyl group,
R² means hydrogen or a -COOH group, wherein the -COOH group also being able to be present as a salt with a physiologically acceptable cation,
R³ means hydrogen or a C₁-C₄ alkyl group,
R⁴ means hydrogen, a C₁-C₄ alkyl group, an amino group or a group -CO-R⁶, in which R⁶ means a C₁-C₄ alkyl group, and
R⁵ means one of the groups mentioned under R⁴,
or a physiologically acceptable salt of these compounds with an organic or inorganic acid, and
derivatives of 5,6-dihydroxyindoline of formula (II), 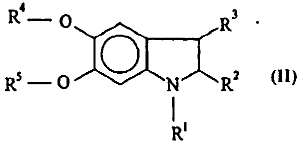 wherein, independently of one another,
R¹ means hydrogen, a C₁-C₄ alkyl group or a C₁-C₄ hydroxyalkyl group,
R² means hydrogen or a -COOH group, wherein the -COOH group also being able to be present as a salt with a physiologically acceptable cation,
R³ means hydrogen or a C₁-C₄ alkyl group,
R⁴ means hydrogen, a C₁-C₄ alkyl group, an amino group or a group -CO-R⁶, in which R⁶ means a C₁-C₄ alkyl group, and
R⁵ means one of the groups mentioned under R⁴,
or a physiologically acceptable salt of these compounds with an organic or inorganic acid;
a xanthine derivative as bioactive substance, wherein the xanthine derivative is caffeine; and at least one direct dye.

2. Cosmetic formulation according to claim 1, wherein the compound of formula (I) is selected from 5,6-dihydroxyindole, N-methyl-5,6-dihydroxyindole, and their physiologically acceptable salts.

3. Cosmetic formulation according to claim 1, wherein the compound of formula (II) is selected from 5,6-dihydroxyindoline, N-methyl-5,6-dihydroxyindoline, and their physiologically acceptable salts.

4. Cosmetic formulation according to any of the claims 1-3, wherein the direct dye is selected from the group consisting of 2-amino-6-chloro-4-nitrophenol, 1,4-bis-(β-hydroxyethyl)-amino-2-nitrobenzene, Basic Yellow 57, Basic Red 76, Basic Brown 16, Basic Brown 17, Basic Blue 99, HC Blue 2, HC Blue 12, HC Blue 16 and Acid Violet 43, preferably HC Blue 2.

5. Non-therapeutic use of the cosmetic formulation according to any of the preceding claims for the repigmentation of keratin fibres, in particular human hair.

6. Use according to claim 5 for the repigmentation of human hair, wherein the formulation is distributed in wet or dry hair and rinsed out again after a residence time of between 1 and 30 minutes.

7. Use according to claim 5 or claim 6 for the repigmentation of human hair, wherein the hair is slowly and gently permanently coloured and at the same time the keratinocytes in the hair roots are stimulated so that they once again increasingly produce natural pigment.

8. Non-therapeutic method for the repigmentation of keratin fibres, in particular human hair, wherein the cosmetic formulation according to any of claims 1-4 is applied to the hair and the colouring subsequently develops.

## Revendications

1. Formulation cosmétique contenant un précurseur de colorant choisi dans le groupe constitué par
les dérivés du 5,6-dihydroxyindole de formule (I) 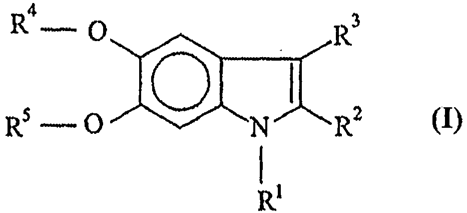 dans laquelle, indépendamment les uns des autres,
R¹ représente l'hydrogène, un groupe alkyle en C₁ à C₄ ou un groupe hydroxyalkyle en C₁ à C₄,
R² représente l'hydrogène ou un groupe -COOH, le groupe -COOH pouvant également se présenter sous forme de sel avec un cation physiologiquement acceptable,
R³ représente l'hydrogène ou un groupe alkyle en C₁ à C₄,
R⁴ représente l'hydrogène, un groupe alkyle en C₁ à C₄, un groupe amino ou un groupe -CO-R⁶ dans lequel R⁶ représente un groupe alkyle en C₁ à C₄, et
R⁵ représente l'un des groupes mentionnés en R⁴,
ou un sel physiologiquement acceptable de ces composés avec un acide organique ou inorganique, et
les dérivés de la 5,6-dihydroxyindoline de formule (II), 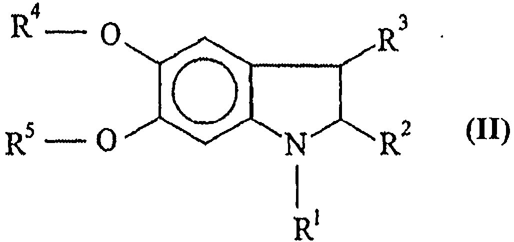 dans laquelle, indépendamment les uns des autres,
R¹ représente l'hydrogène, un groupe alkyle en C₁ à C₄ ou un groupe hydroxyalkyle en C₁ à C₄,
R² représente l'hydrogène ou un groupe -COOH, le groupe -COOH pouvant également se présenter sous forme de sel avec un cation physiologiquement acceptable,
R³ représente l'hydrogène ou un groupe alkyle en C₁ à C₄,
R⁴ représente l'hydrogène, un groupe alkyle en C₁ à C₄, un groupe amino ou un groupe -CO-R⁶ dans lequel R⁶ représente un groupe alkyle en C₁ à C₄, et
R⁵ représente l'un des groupes mentionnés en R⁴,
ou un sel physiologiquement acceptable de ces composés avec un acide organique ou inorganique,
un dérivé de la xanthine comme substance bioactive, le dérivé de la xanthine étant la caféine ; et
au moins un colorant montant directement sur la fibre.

2. Formulation cosmétique selon la revendication 1,
dans laquelle le composé de formule (I) est choisi parmi le 5,6-dihydroxyindole, le N-méthyl-5,6-dihydroxyindole et leurs sels physiologiquement acceptables.

3. Formulation cosmétique selon la revendication 1,
dans laquelle le composé de formule (II) est choisi parmi la 5,6-dihydroxyindoline, la N-méthyl-5,6-dihydroxyindoline et leurs sels physiologiquement acceptables.

4. Formulation cosmétique selon l'une des revendications 1 à 3,
dans laquelle le colorant montant directement sur la fibre est choisi dans le groupe constitué par 2-amino-6-chloro-4-nitrophénol, 1,4-bis-(β-hydroxyéthyl)-amino-2-nitrobenzène, Basic Yellow 57, Basic Red 76, Basic Brown 16, Basic Brown 17, Basic Blue 99, HC Blue 2, HC Blue 12, HC Blue 16 et Acid Violet 43, de préférence HC Blue 2.

5. Utilisation non thérapeutique de la formulation cosmétique selon l'une des revendications précédentes pour la re-pigmentation des fibres kératiniques, en particulier des cheveux humains.

6. Utilisation selon la revendication 5 pour la re-pigmentation des cheveux humains,
dans laquelle la formulation est répartie sur les cheveux humides ou secs et est rincée après un temps de séjour compris entre 1 et 30 minutes.

7. Utilisation selon la revendication 5 ou la revendication 6 pour la repigmentation des cheveux humains,
dans laquelle les cheveux sont teints de manière permanente, lentement et en douceur, et les kératinocytes dans les racines des cheveux sont stimulés en même temps afin qu'ils produisent à nouveau davantage de pigments naturels.

8. Procédé non thérapeutique pour la re-pigmentation des fibres kératiniques, en particulier des cheveux humains,
dans lequel la formulation cosmétique selon l'une des revendications 1 à 4 est appliquée sur les cheveux, ce qui entraîne la coloration.
